# EUROPÄISCHE PATENTSCHRIFT

(11) **EP 0 761 246 B1**
(45) Veröffentlichungstag und Bekanntmachung des Hinweises auf die Patenterteilung: **21.11.2001**
(21) Anmeldenummer: 96250169.8
(22) Anmeldetag: 07.08.1996
(51) Int. Cl.: A61M 3/02

(54) **Vorrichtung zum Aufbringen von Medikamentenflüssigkeit auf Schleimhäute in Körperhöhlungen**
Application device for liquid drug onto the mucous membrane of body cavities
Dispositif pour l'application d'un médicament liquide sur la muceuse des cavités corporelles

(30) Priorität: 10.08.1995 DE 19530879
(43) Veröffentlichungstag der Anmeldung: 12.03.1997
(73) Patentinhaber: Schülke & Mayr GmbH, 22851 Norderstedt (DE)
(72) Erfinder: Grothoff, Hans, 44225 Dortmund (DE); Harke, Hans-Peter, Dr., 22419 Hamburg (DE); Hennig, Frank, 24610 Trappenkamp (DE); Neumann, Gerd, Dr., 22145 Hamburg (DE)
(74) Vertreter: UEXKÜLL & STOLBERG

(56) Entgegenhaltungen:
- EP-A- 0 363 519
- US-A- 2 974 666
- US-A- 3 731 676
- US-A- 3 783 867
- US-A- 5 248 304

## Beschreibung

Die Erfindung betrifft eine Vorrichtung zum Aufbringen von Medikamentenflüssigkeit auf Schleimhäute im Inneren von Körperhöhlungen, insbesondere im Oral-, Vaginal- und Analbereich, für alle vorbeugenden und therapeutischen Anwendungen von Flüssigkeiten.

Bei der Behandlung dieser Körperzonen ist es von herausragender Bedeutung, daß diese einfach und ohne besonderen Aufwand sowohl im privaten Bereich als auch in Arztpraxen und Krankenhäusern durchgeführt werden kann. Ein weiterer wesentlicher Punkt liegt darin, Medikamentenflüssigkeit möglichst umfassen und vollflächig aufzubringen, damit der gesamte Schleimhautbereich auch bei geringer Dosierung erfaßt wird.

Für eine gezielte Therapie, z.B. im Vaginalbereich, ist eine umfassende Benetzung des zu behandelnden Bereichs von wesentlicher Bedeutung. Beispielsweise werden sexuell übertragbare Infektionen im Vaginalbereich von Gynäkologen mit spezifischen Chemotherapeutika behandelt. Der Gynäkologe kann bei derartigen Behandlungen die Medikamentenflüssigkeit über Tupfer, Sprühkopf oder als Spülung applizieren. Wünschenswert ist darüber hinaus aber oft eine begleitende Applikation durch die Patienten selbst, die nichtstationär wiederholt vorgenommen werden kann. Für eine derartige Selbstmedikation stehen bisher keine geeigneten Vorrichtungen zur Verfügung.

Es ist eine Vorrichtung zur Spülung des Vaginalbereichs bekannt, die eine Faltenbalgflasche mit einem darauf aufschraubbaren Einführrohr mit mehreren Öffnungen an der Spitze besitzt. Mit dieser Vorrichtung sind Spülungen, d.h. die Aufbringung von großen Flüssigkeitsmengen auf den betroffenen Schleimhautbereich möglich, nicht jedoch die Aufbringung geringer Dosen bei gleichztig vollflächiger Verteilung auf dem betroffenen Bereich. Ein weiterer Nachteil besteht darin, daß, da nur große Flüssigkeitsmengen zur Anwendung gebracht werden können und diese zum größten Teil sofort wieder abfließen müssen, die Applikation nur unter speziellen Vorkehrungen vorgenommen werden kann, z.B. in einer nicht mit Wasser gefüllten Badewanne, damit das austretende Präparat gefahrlos abfließen kann.

Ferner sind mit der bekannten Vorrichtung nur einmalige Spülungen unbedenklich möglich, da nach Einbringen des Einführrohrs in den Vaginalbereich und Zusammendrücken der Faltenbalgflasche nicht verläßlich sicherstellbar ist, daß vor Entfernen der Vorrichtung durch unbeabsichtigte Ausdehnung der Faltenbalgflasche ein Rücksaugeffekt auftritt, was das Eintreten von Körperflüssigkeit in die Vorrichtung zur Folge hat und eine Wiederverwendung der Vorrichtung ohne eingehende Sterilisation höchst bedenklich macht.

Aus EP-A-0 363 519 ist eine vaginale Spüleinrichtung mit den Merkmalen des Oberbegriffs des Anspruchs 1 bekannt. Die Einrichtung hat eine manuell steuerbare Pumpeneinrichtung an einem Medikamentenvorratsbehälter zur Abgabe von Medikamentenflüssigkeit unter Druck aus einem Auslaß, ein mit dem Auslaß verbundenes Einführrohr mit hindurchgehender Längsbohrung und einen Verteilerkörper am gegenüberliegenden Ende des Einführrohrs, der nach Außenform und Volumen an die zu behandelnde Körperhöhlung angepaßt ist und der über seine Außenfläche verteilt eine Vielzahl von Auslaßöffnungen aufweist, die über einen Innenraum des Verteilerkörpers mit dem Einführrohr kommunizieren, so daß unter Druck zugeführte Medikamentenflüssigkeit aus den Auslaßöffnungen über die Oberfläche des Verteilerkörper verteilt ausgegeben wird.

Es ist Aufgabe der vorliegenden Erfindung, eine Vorrichtung zum Aufbringen von Medikamentenflüssigkeit in Körperhöhlungen zu schaffen, die eine hygienische und vollflächige Aufbringung der Medikamentenflüssigkeit ermöglicht.

Zur Lösung dieser Aufgabe dient die Vorrichtung mit den Merkmalen des Patentanspruchs 1. Vorteilhafte Ausgestaltungsformen der Erfindung sind in den Unteransprüchen aufgeführt.

Um eine feine Versprühung und Verteilung der Medikamentenflüssigkeit zu erreichen, weisen die Auslaßöffnungen einen geringen Durchmesser von im Mittel weniger als 200 µm auf, vorteilhaft liegt der Durchmesser im Bereich zwischen 5 µm und 50 µm. Derart feine Auslaßöffnungen können z.B. durch Laserbohren eines Kunststoffhohlkörpers gebildet werden. Alternativ kann der Verteilerkörper aus mikroporösem Material, z.B. aus offenzelligem Schaumstoff, hergestellt werden, um eine feine Verteilung der Medikamentenflüssigkeit über die Mikroporen zu erreichen. Ferner ist eine Rückströmungssicherung vorgesehen, so daß Medikamentenflüssigkeit nur ausgegeben werden kann, aber kein Rücksaugen von Flüssigkeiten in den Verteilerkörper hinein auftreten kann. Dies wird durch eine Einwegventileinrichtung realisiert.

In einer bevorzugten Ausführungsform werden die Auslaßöffnungen des Verteilerkörpers dadurch gebildet, daß eine Mehrzahl von Bohrungen zum Innenraum des Verteilerkörpers vorgesehen sind, in welche jeweils ein Einpreßkörper fest eingesteckt ist, der die Querschnittsfläche der jeweiligen Bohrung reduziert und in eine Mehrzahl kleinerer Auslaßöffnungen unterteilt, so daß eine fein versprühte Ausgabe der Medikamentenflüssigkeit erreicht wird, ohne Bohrungen mit extrem kleinem Durchmesser herstellen zu müssen.

In einer vorteilhaften Ausführungsform wird die Pumpeneinrichtung durch eine fingerdruckbetriebene Druckaufbau-Feinzerstäuberpumpe gebildet, die eine Überdruckfunktion derart haben, daß erst nach Überschreiten einer konstruktiv vorgegebener Kompressionsschwelle der Flüssigkeitsauslaß freigegeben wird, wobei die vorgegebene Kompressionsschwelle in typischen Fällen zwischen 2 und 20 bar liegt. Dadurch ist einerseits sichergestellt, daß die Medikamentenflüssigkeit mit relativ hohem Druck in den Verteilerkörper eintritt und gleichmäßig über den Auslaßöffnungen ausgestoßen wird. Andererseits wird auf einfache Weise verhindert, daß zu irgendeinem Zeitpunkt Unterdruck im Verteilerkörper entstehen könnte, so daß Flüssigkeit aus der Umgebung in den Verteilerkörper zurückgesaugt werden könnte. Dadurch ist die Sterilhaltung des Verteilerkörpers wesentlich einfacher zu gewährleisten und eine Mehrfachverwendung bei derselben Patientin unbedenklich. Ferner können solche Zerstäuberpumpen ausgewählt werden, die pro Betätigung des Drückers eine sehr geringe Flüssigkeitsmenge förden, wobei ein typischer Wert im Bereich von 0,1 bis 2 ml liegen kann. Dies vereinfacht eine feindosierte Abgabe und Anwendung von Medikamentenflüssigkeit.

Die Erfindung wird im folgenden anhand von Ausführungsbeispielen in den Zeichnungen beschrieben, in denen:
- Figur 1: eine teilweise geschnittene Seitenansicht einer Vorrichtung zum Aufbringen von Medikamentenflüssigkeit in Körperhöhlungen zeigt;
- Figur 2: eine teilweise geschnittene Teilansicht der Vorrichtung aus Figur 1 ist, die eine Pumpeneinrichtung, ein Einführrohr und einen Verteilerkopf zeigt;
- Figur 3: eine teilweise geschnittene Ansicht einer alternativen Ausführungsform des Verteilerkörpers zeigt;
- Figur 4: eine Schnittansicht einer weiteren alternativen Ausführungsform des Verteilerkörpers zeigt;
- Figur 5: eine Ansicht einer weiteren Ausführungsform des Verteilerkörpers zeigt; und
- Figur 6: eine vergrößerte Detailansicht des Oberflächenbereichs einer Auslaßöffnung auf dem Verteilerkörper aus Figur 6 zeigt.

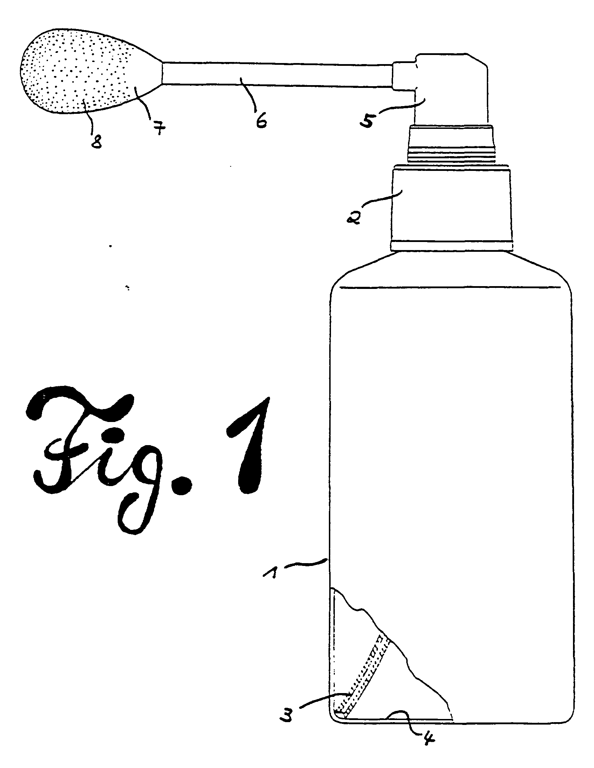

Figur 1 zeigt eine Gesamtdarstellung einer Vorrichtung zum Aufbringen von Medikamentenflüssigkeit in Körperhöhlungen. Die Vorrichtung weist eine Pumpeneinrichtung 2, die als fingerdruckbetätigte Zerstäuberpumpe ausgebildet ist, und einen Vorratsbehälter 1 auf, an dem die Pumpeneinrichtung angebracht ist. An einem an dem Drücker 5 der Pumpeneinrichtung 2 liegenden Auslaß der Pumpe ist ein Einführrohr 6 mit hindurchgehender Längsbohrung angebracht. Am anderen Ende des Einführrohrs 6 ist ein Verteilerkörper 7 angebracht, dessen Innenraum mit der Längsbohrung des Einführrohrs 6 kommuniziert und der eine Vielzahl von Auslaßöffnungen 8 in Verbindung mit dem Innenraum aufweist. Die Pumpeneinrichtung 2 ist mit einem Steigrohr 3 versehen, das bis zum Boden 4 des Vorratsbehälters 1 hinunterreicht, damit eine vollständige Entleerung des Vorratsbehälters 1 möglich ist.

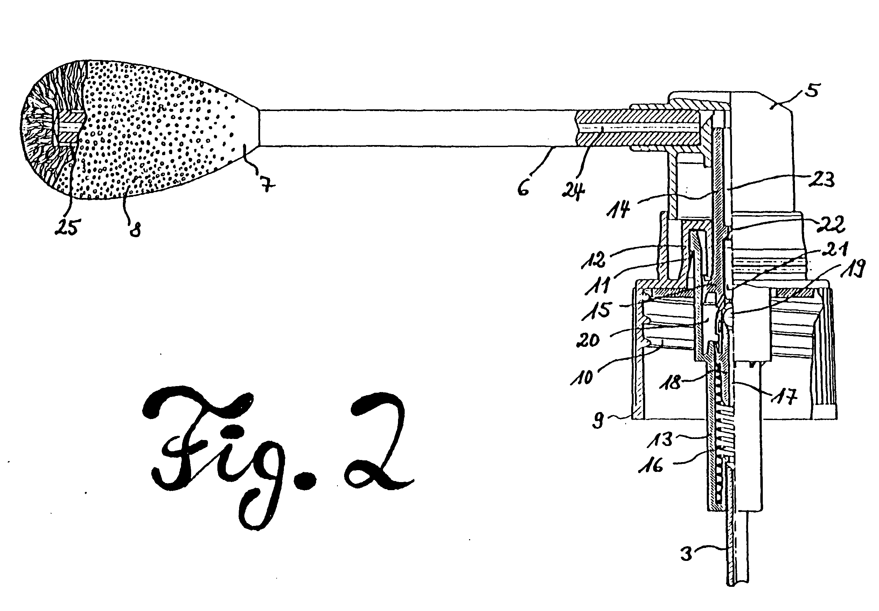

In Figur 2 ist zur Verdeutlichung beispielhaft der Aufbau einer fingerdruckbetätigten Zerstäuberpumpe im Schnitt dargestellt. Der Schraubkörper 9 dient mit seinen Gewindegängen 10 zu Befestigung auf einem Hals des Vorratsbehälters 1 mit dazu passenden Gewinde (nicht gezeigt). Durch eine Schnappverbindung 11 ist im Dom 12 des Schraubkörpers 9 ein Zylinder 13 befestigt, in dem wiederum das Steigrohr 3 durch Preßsitz gehalten wird. Der Schaft 14 eines Arbeitskolbens 15 ragt durch eine Bohrung über den Dom 12 heraus. Auf dem Schaft 14 sitzt der Drücker 5. Die Flüssigkeit wird beim durch die vorgespannte Feder 16 bewirkten Rückhub des Drückers 5 durch Unterdruck über das Steigrohr 3 und eine Innenbohrung 17 eines Steuerkolbens 18 unter Anheben einer Rückschlagkugel 19 in den zwischen dem Arbeitskolben 15 und dem Steuerkolben 18 im oberen, durchmessergrößeren Bereich des Zylinders 13 angeordneten Kompressionsraum 20 gefördert.

Bei der Betätigung des Drückers 5 eilt der Steuerkolben 18 nach dem Differentialkolbenprinzip dann gegenüber dem Arbeitskolben 15 vor, sobald der auf den Steuerkolben 18 einwirkende Flüssigkeitsdruck den Gegendruck der Feder 16 übersteigt, und öffnet durch Rückziehen des mit dem Steuerkolben 18 fest verbundenen Absperrstiftes 21 die im Arbeitskolben 15 befindliche Durchgangsbohrung 22. Die im Kompressionsraum 20 befindliche vorgespannte Flüssigkeit strömt dann über die Verbindungsbohrung 23 erst in den Drücker 5 und von dort durch die im Einführrohr 6 verlaufende Längsbohrung 24 in den Innenraum des Verteilerkörpers 7 ein.

In dem in Figur 2 dargestellten Ausführungsbeispiel ist der tropfenförmige Verteilerkörper 7 aus einem mikroporösem Material hergestellt, beispielsweise aus offenzelligem Schaumstoff oder als Sinterteil. Bedingt durch die relativ große Gesamtoberfläche der Poren ist der sich durch die Pumpeneinrichtung aufbauende Druck im Innenraum des Verteilerkörpers 7 relativ gering, so daß die Flüssigkeit in Form eines über die gesamte Außenfläche des Verteilerkörpers gleichmäßig verteilten Films austritt. Diese Ausführungsform ist damit besonders zur Behandlung von durch den Verteilerkörper 7 unmittelbar und vollständig kontaktierbaren Schleimhautbereichen geeignet.

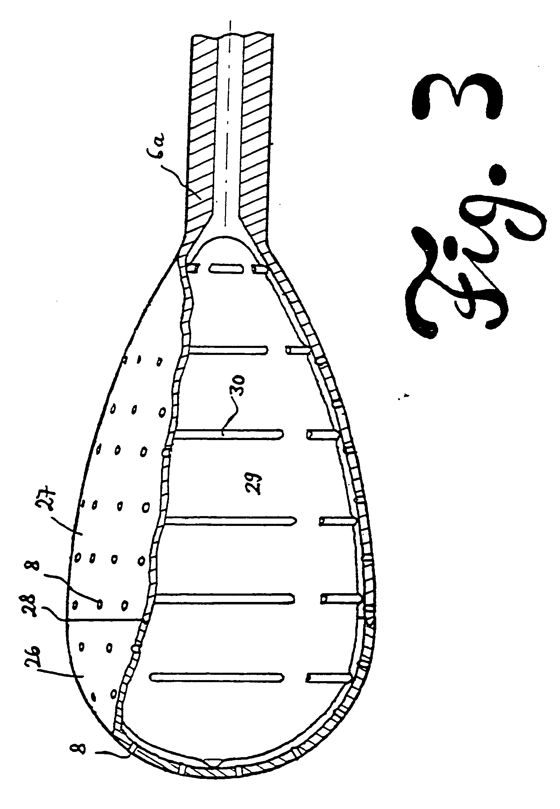

Eine vorteilhafte Ausführungsform des Verteilerkörpers, die sich durch eine einfache Variierbarkeit der Ausbringungscharakteristika der Flüssigkeit auszeichnet, ist in Figur 3 dargestellt. Der Verteilerkörper besteht aus zwei dicht ineinandergefügten Hälften 26, 27, die mit einer Vielzahl von Auslaßöffnungen 8 versehen sind. Die Fügestelle 28 ist im Bereich des größten Durchmessers des tropfenförmigen Verteilerkörpers angeordnet und kann als Schnapp-, Schweiß- oder Klebeverbindung ausgeführt sein. Die hintere Hälfte des Verteilerkörpers kann, wie im vorangegangenen Beispiel, auf das Einführrohr 6 aufgesteckt sein, vorteilhafter ist es jedoch, den Einführrohrendbereich 6a, wie in Figur 3 dargestellt, an die hintere Hälfte 27 des Verteilerkörpers direkt anzuformen.

Damit der Innenraum des Verteilerkörpers nur ein geringes Totvolumen aufweist, ist er mit einem Füllkörper 29 versehen, der über Stege 30 in gleichmäßigem Abstand zur Innenwand der Verteilerkörperhälften liegt. Der Füllkörper 29 ist vorteilhaft entweder aus einem flüssigkeitsdichten Hohlkörper oder einem geschlossenzelligen Schaumstoff gefertigt, um das Gesamtgewicht der Vorrichtung zu minimieren.

Die Austrittsöffnungen 8 werden zweckmäßigerweise durch Laserbohren erzeugt. Über Anzahl und Größe der Öffnungen läßt sich die Abgabeweise der Medikamentenflüssigkeit einstellen, von der Bildung von weitreichenden Einzelstrahlen bis hin zur filmartigen, flächigen Benetzung des Verteilerkörpers, je nach den Erfordernissen des Anwendungszwecks. So ergibt sich beispielsweise bei einer Lochgröße von 20 µm und einer Lochzahl von 100 bei einer niedrig viskosen Flüssigkeit eine ausgeprägt strahlförmige Ausgabeweise, während eine Verdoppelung des Öffnungsdurchmessers auf 40 µm oder eine Erhöhung der Lochzahl auf 400 zu einer filmartigen Benetzung führt. Durch entsprechende Auswahl verschiedener Öffnungsdurchmesser lassen sich auch kombinierte Effekte herstellen. So führt z.B. die Kombination von Öffnungen von 5 µm und 20 µm Durchmesser bei entsprechend abgestimmter Lochzahl zu einem Verteilerkörper, der sowohl eine strahlartige Flüssigkeitsabgabe als auch gleichzeitig eine filmartige Oberflächenbenetzung bewirkt.

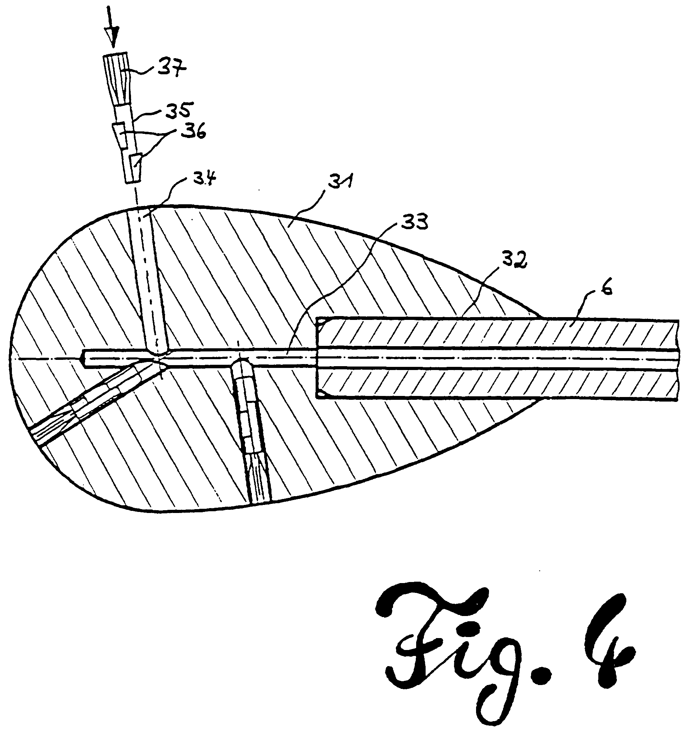

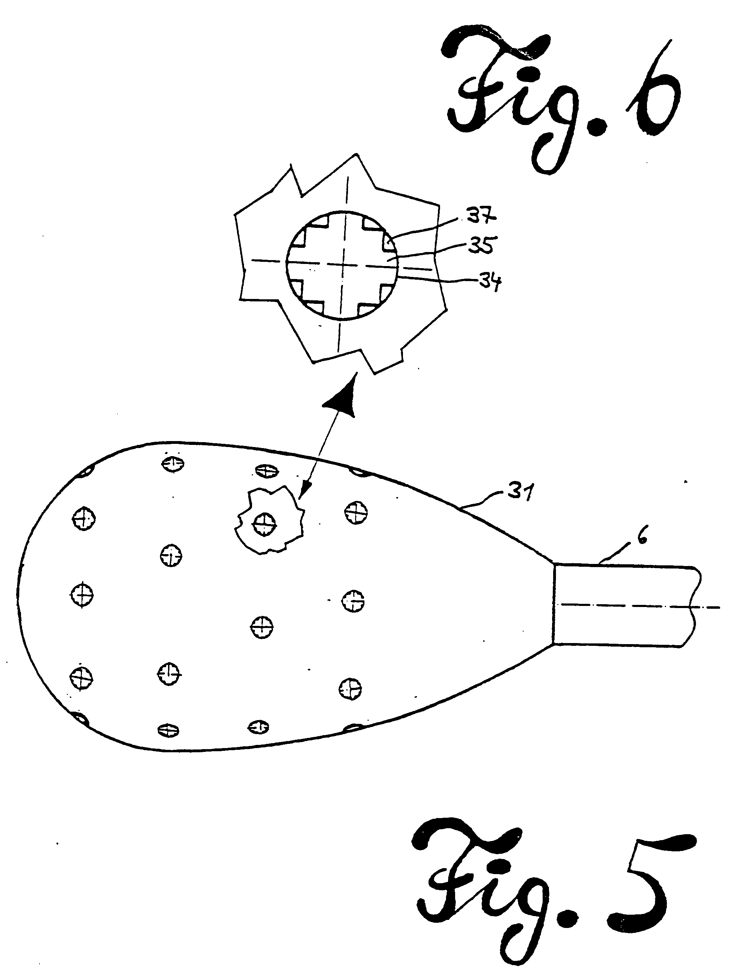

Allerdings erfordert die zuvor geschilderte Ausführungsform einigen herstellungstechnischen Aufwand, da eine Laserbohreinrichtung und weitere Werkzeuge und Formen erforderlich sind. Die Ausführungsform gemäß den Figuren 4 bis 6 ist dagegen bedeutend einfacher herzustellen. Der dargestellte Verteilerkörper 31 ist in diesem Fall aus Vollmaterial, vorzugsweise Kunststoff, spanabhebend auf Drehautomaten gefertigt, wobei üblicherweise von Stangenmaterial ausgegangen wird. Nach dem Bohren der Aufnahmebohrung 32 für das Einführrohr 6 und der anschließenden Zentralbohrung 33 wird die gewünschte tropfenförmige Außenkontur angedreht und der Körper abgestochen. Die Bohrungen 34 werden zweckmäßigerweise voll automatisch auf Ein- oder Mehrspindelbohrautomaten eingebracht und stehen mit der Zentralbohrung 33 in Verbindung. In jede Bohrung 34 wird nun ein Einpreßkörper 35 eingedrückt, der gegenüber der Bohrung 34 ein Übermaß aufweist und dadurch sicher in der Bohrung 34 sitzt. Eine zusätzliche Sicherung kann durch die Anordnung von Haltekrallen 36, die am unteren Teil des Schaftes angeordnet sind, erreicht werden.

Die Einpreßkörper 35 weisen im vorderen Bereich Ausnehmungen in Form von axial verlaufenden Nuten 37 auf, deren geometrischer Querschnitt sich vornehmlich aus den technischen Gegebenenheiten des Herstellungsverfahrens ergibt. Die in der Aufsicht von Figur 6 dargestellte dreieckige Form der Nuten resultiert aus den Herstellungsmöglichkeiten im Kunststoff-Spritzgußverfahren. Die axial verlaufenden Nuten bilden dann die in der Bohrung 34 verbleibenden Austrittskanäle. Durch die Nuten 37 an dem Einpreßkörper 35 wird die Querschnittsfläche jeder Bohrung 34 reduziert und eine Mehrzahl von deutlich kleineren Auslaßöffnungen 8 gebildet. Auf diese Weise können auch bei mechanisch einfach herstellbaren Bohrungen mit relativ großem Durchmesser sehr feine Auslaßöffnungen realisiert werden.

## Patentansprüche

1. Vorrichtung zum Aufbringen von Medikamentenflüssigkeit auf Schleimhäute im Inneren von Körperhöhlungen, mit:
einer manuell steuerbaren Pumpeneinrichtung (2) an einem Medikamentenvorratsbehälter (1) zur Abgabe von Medikamentenflüssigkeit unter Druck aus einem Auslaß (5),
einem mit dem Auslaß (5) verbundenen Einführrohr (6) mit hindurchgehender Längsbohrung (24), und
einem mit dem gegenüberliegenden Ende des Einführrohrs (6) verbundenen Verteilerkörper (7), der nach Außenform und Volumen an die zu behandelnde Körperhöhlung angepaßt ist und der über seine Außenfläche verteilt eine Vielzahl von Auslaßöffnungen (8; 34) aufweist, die über einen Innenraum des Verteilerkörpers mit dem Einführrohr (6) kommunizieren, so daß unter Druck zugeführte Medikamentenflüssigkeit aus den Auslaßöffnungen (8; 34) über die Oberfläche des Verteilerkörper (7) verteilt ausgegeben wird,
**dadurch gekennzeichnet,**
**daß** die Auslaßöffnungen (8) des Verteilerkörpers einen mittleren Durchmesser von weniger als 200 µm haben und daß eine Einwegventileinrichtung vorhanden ist, so daß Rückströmung aus dem Verteilerkörper entgegen der Pumprichtung unterbunden wird.

2. Vorrichtung nach Anspruch 1, wobei die Pumpeneinrichtung (2) durch eine fingerdruckbetriebene Druckaufbau-Feinzerstäuberpumpe gebildet wird.

3. Vorrichtung Anspruch 1, wobei die Pumpeneinrichtung durch eine handbetriebene, rückstromgesicherte Dispenser- oder Zerstäubereinheit gebildet wird.

4. Vorrichtung nach Anspruch 1, wobei die Pumpeneinrichtung durch eine unter Treibgasdruck stehende Druckzerstäuberpakkung gebildet wird.

5. Vorrichtung nach einem der vorhergehenden Ansprüche, wobei die Pumpeinrichtung die Medikamentenflüssigkeit unter einem Druck von 2 bis 20 bar ausgibt.

6. Vorrichtung nach einem der vorhergehenden Ansprüche, wobei der Verteilerkörper (7) durch einen Kunststoffhohlkörper gebildet ist, in dessen Außenwand eine Vielzahl von Auslaßöffnungen (8) mit Durchmessern im Bereich zwischen 5 µm und 50 µm gebildet sind.

7. Vorrichtung nach einem der vorhergehenden Ansprüche, wobei der Verteilerkörper aus zwei dichtend ineinandergefügten Hälften aufgebaut ist.

8. Vorrichtung nach einem der vorhergehenden Ansprüche, wobei im Innernraum des Verteilerkörpers ein flüssigkeitsdichter Füllkörper so befestigt ist, daß Durchflußwege vom Eintritt des Einführrohrs zu allen Austrittsöffnungen frei bleiben.

9. Vorrichtung nach Anspruch 8, wobei der Füllkörper aus Schaumstoff mit geschlossener Oberfläche besteht oder als Hohlkörper ausgebildet ist.

10. Vorrichtung nach einem der vorhergehenden Ansprüche, wobei die Auslaßöffnungen (37) des Verteilerkörpers durch eine Mehrzahl von Bohrungen (34) zum Innnenraum gebildet werden, in welche jeweils ein Einpreßkörper (35) fest eingesteckt ist, der die Öffnungsfläche der jeweiligen Bohrung (34) reduziert und in eine Mehrzahl kleinerer Auslaßöffnungen (37) unterteilt.

11. Vorrichtung nach einem der Ansprüche 1 bis 5, wobei der Verteilerkörper aus mikroporösem Material, vorzugsweise aus offenzelligem Schaumstoff, hergestellt ist, wobei die Mikroporen die Auslaßöffnungen (8) bilden.

12. Vorrichtung nach einem der vorhergehenden Ansprüche, wobei der Verteilerkörper (7) und das Einführrohr (6) direkt'miteinander verbunden sind.

13. Vorrichtung nach Anspruch 12, wobei der Verteilerkörper (7) und das Einführrohr (6) einstückig ausgebildet sind.

14. Vorrichtung nach einem der vorhergehenden Ansprüche, wobei die Pumpeneinrichtung und das Einführrohr (6) direkt miteinander verbunden sind.

15. Vorrichtung nach einem der Ansprüche 1 bis 13, wobei die Pumpeneinrichtung und das Einführrohr (6) über ein flexibles Schlauchstück miteinander verbunden sind.

## Claims

1. Device for the application of liquid medication to mucous membranes inside body cavities, with:
a manually controllable pump device (2) on a medication reservoir (1) for dispensing liquid medication under pressure from an outlet (5),
a delivery tube (6), connected with the outlet (5), with longitudinal bore (24) throughout, and
a distributor body (7), connected to the opposite end of the delivery tube (6), whose external form and volume are adapted to the body cavity to be treated and which has, distributed over its external surface, a large number of outlet openings (8; 34), which are connected to the delivery tube (6) via an internal space in the distributor body, so that liquid medication delivered under pressure from the outlet openings (8, 34) is distributed over the surface of the distributor body (7),
**characterized in that**,
the outlet openings (8) of the distributor body have an average diameter of less than 200 µm and that there is a one-way valve device, preventing reverse flow from the distributor head against the pumping direction.

2. Device according to Claim 1, in which the pump device (2) is formed by a pressure build-up atomizing pump operated by finger pressure.

3. Device according to Claim 1, in which the pump device is formed by a manually operated, dispensing or atomizing unit protected against reverse flow.

4. Device according to Claim 1, in which the pump device is formed by a pressure atomizer pack under pressurised propellant gas.

5. Device according to any of the preceding claims, in which the pump device dispenses the liquid medication at a pressure of 2 to 20 bar.

6. Device according to any of the preceding claims, in which the distributor body (7) is formed from a hollow plastic body, in whose outer wall are formed a large number of outlet openings (8) with diameters ranging between 5 µm and 50 µm.

7. Device according to any of the preceding claims, in which the distributor body is constructed of two halves joined together to form a seal.

8. Device according to any of the preceding claims, in which, in the internal space of the distributor body, a liquid-proof filling body is fitted in such a way that flow paths remain clear from the delivery tube inlet to all outlet openings.

9. Device according to Claim 8, in which the filling body consists of a foam with a closed surface or is formed as a hollow body.

10. Device according to any of the preceding claims, in which the outlet openings (37) of the distributor body are formed by a number of bores (34) into the internal space, into each of which is firmly pressed a plug (35), which reduces the open-area of each bore (34) and divides it into a number of smaller outlet openings (37).

11. Device according to any of Claims 1 to 5, in which the distributor body is made from microporous material, preferably from open-celled foam material, in which the micropores form the outlet openings (8).

12. Device according to any of the preceding claims, in which the distributor body (7) and the delivery tube (6) are directly connected to each other.

13. Device according to any of the preceding claims, in which the distributor body (7) and the delivery tube (6) are integrally formed.

14. Device according to any of the preceding claims, in which the pump device and the delivery tube (6) are directly connected to each other.

15. Device according to any of Claims 1 to 13, in which the pump device and the delivery tube (6) are connected to each other via a length of flexible tubing.

## Revendications

1. Dispositif pour l'application de médicaments liquides sur des muqueuses à l'intérieur de cavités du corps, comprenant:
- un dispositif de pompage (2) commandé manuellement monté sur un réservoir à médicament liquide (1), aux fins de délivrer du liquide médicament liquide sous pression à travers une sortie (5),
- une canule (6) liée à la sortie (5), munie d'un trou longitudinal (24) traversant,
- un élément distributeur (7), lié à l'extrémité opposée de la canule (6), dont la forme extérieure et le volume sont adaptés à la cavité du corps à traiter et qui présente, répartis sur sa surface extérieure, une pluralité d'orifices de sortie (8; 34) qui communiquent avec la canule (6) par l'intermédiaire d'une chambre intérieure du distributeur, de telle sorte que le médicament liquide arrivant sous pression soit réparti par les orifices de sortie (8; 34) à la surface de l'élément distributeur (7), **caractérisé en ce que**
les orifices de sortie (8) de l'élément distributeur ont un diamètre moyen inférieur à 200 µm et **en ce qu'**un système de clapet anti-retour est prévu, qui empêche un reflux de l'élément distributeur dans la direction opposée à la direction de pompage.

2. Dispositif selon la revendication 1, dans lequel le dispositif de pompage (2) est formé d'une pompe-pulvérisateur de mise en pression, actionnée par le doigt.

3. Dispositif selon la revendication 1, dans lequel le dispositif de pompage (2) est formé d'une unité doseuse ou d'une unité pulvérisatrice manuelle, à sécurité anti-reflux.

4. Dispositif selon la revendication 1, dans lequel le dispositif de pompage (2) est formé d'un emballage pulvérisateur mû par la pression d'un gaz propulseur.

5. Dispositif selon une des revendications précédentes, dans lequel le dispositif de pompage délivre le médicament liquide sous une pression comprise dans une plage allant de 2 à 20 bars.

6. Dispositif selon une des revendications précédentes, dans lequel l'élément distributeur (7) est formé d'un corps creux en matière plastique, dans la paroi duquel sont formés une pluralité d'orifices de sortie (8) avec des diamètres compris dans une plage allant de 5 µm à 50 µm.

7. Dispositif selon une des revendications précédentes, dans lequel l'élément distributeur est formé de deux moitiés assemblées de manière étanche.

8. Dispositif selon une des revendications précédentes, dans lequel un corps de remplissage étanche aux liquides est fixé à l'intérieur de la chambre intérieure de l'élément distributeur, de manière telle que des voies d'écoulement entre l'entrée de la canule et tous les orifices de sortie restent dégagées.

9. Dispositif selon la revendication 8, dans lequel le corps de remplissage est formé de mousse à cellules fermées ou est conformé en corps creux.

10. Dispositif selon une des revendications précédentes, dans lequel les orifices de sortie (37) de l'élément distributeur sont formés d'une pluralité de trous (34) communiquant avec la chambre intérieure, dans lesquels est emmanché chaque fois un insert (35) qui réduit la section d'ouverture du trou concerné (34) et la divise en une pluralité d'orifices de sortie (37) plus petits.

11. Dispositif selon une des revendications 1 à 5, dans lequel l'élément distributeur est formé d'un matériau micro-poreux, de préférence de mousse à cellules ouvertes, les micro-pores formant les orifices de sortie (8).

12. Dispositif selon une des revendications précédentes, dans lequel l'élément distributeur (7) et la canule (6) sont liés directement l'un à l'autre .

13. Dispositif selon la revendication 12, dans lequel l'élément distributeur (7) et la canule (6) sont réalisés d'une pièce.

14. Dispositif selon une des revendications précédentes, dans lequel le dispositif de pompage et la canule (6) sont liés directement l'un à l'autre.

15. Dispositif selon une des revendications 1 à 13, dans lequel le dispositif de pompage et la canule (6) sont liés l'un à l'autre par un tronçon de tuyau flexible.
